# EUROPEAN PATENT APPLICATION

(11) **EP 4 574 147 A1**
(43) Date of publication of application: **25.06.2025**
(21) Application number: 23855131.1
(22) Date of filing: 14.08.2023
(51) Int. Cl.: A61K 31/353, A61P 35/00, A61P 35/04, A23L 33/10

(54) **COMPOSITION FOR PREVENTING, AMELIORATING, OR TREATING CANCER COMPRISING STEPPOGENIN AS ACTIVE INGREDIENT**

(30) Priority: 19.08.2022 KR 20220103774
(71) Applicant: Kyungpook National University Industry-Academic Cooperation Foundation, Daegu 41566 (KR)
(72) Inventor: LEE, You Mie, Chilgok-gun, Gyeongsangbuk-do 39855 (KR)
(74) Representative: Nederlandsch Octrooibureau
(86) International application number: PCT/KR2023/012052
(87) International publication number: WO 2024/039164

(57) **Abstract**

The present invention relates to a composition or the like for preventing, ameliorating or treating cancer comprising steppogenin as an active ingredient. More particularly, the steppogenin of the present invention can inhibit the growth of cancer cells by suppressing the expression of HIF-1α, which is overexpressed in cancer cells, and therefore, it is expected to be used for the prevention, amelioration, or treatment of various solid cancer diseases such as lung cancer, liver cancer, and stomach cancer, and also to be usefully utilized for suppressing the malignancy, recurrence, and metastasis of cancer.

## Description

### [Technical Field]

The present invention relates to a composition or the like for preventing, ameliorating or treating cancer comprising steppogenin as an active ingredient.

This invention claims priority to Korean Patent Application No. 10-2022-0103774, filed on August 19, 2022, the entire contents of the specification and drawings of which are incorporated herein by reference.

### [Background Art]

Hypoxia inducible factor 1 (HIF-1) is a heterodimeric transcription factor composed of HIF-1α, which regulates the actual activity of HIF-1, and HIF-1β, which acts as a nuclear transporter. HIF-1α is a transcription factor belonging to the basic-helix-loop-helix-PAS (PER-ARNT-SIM) superfamily. Under normoxia, hydroxylation occurs at residues, Pro594 and Pro402, of the oxygen-degradation domain (ODD), and ODD is degraded by the von Hippel Lindau (VHL)-ubiquitin E3 ligase complex. However, under hypoxia (oxygen ratio below 5%) that mainly occur in various solid cancers, hydroxylation is inhibited, and ODD is not degraded, but moves from the cytoplasm to the nucleus in a dimer state and binds to the hypoxia response element (HRE), thereby inducing the expression of genes involved in angiogenesis, glycolysis, cell growth, and differentiation (Veronica A. et al., Cancer Research, 66(12), 6264-70, 2006; Semenza GL. et al., Nature Review Cancer 3, 721-32, 2003).

Meanwhile, hypoxia in cancer, especially solid cancer, is a common phenomenon, and solid cancer cells adapt to these hypoxia conditions through various genetic changes, making cancer cells more malignant and resistant to anticancer drugs. In fact, hypoxia is known to be a major factor in the malignancy of more than 70% of all cancers in humans (Nature 386, 403, 1997; Oncol. 28, 36-41, 2001, Nat. Med. 6, 1335, 2000; Cancer 97, 1573-1581, 2003).

HIF-1 is known as the most important molecule that regulates the adaptation of cancer cells to hypoxia, and in particular, the amount of HIF-1α protein and the prognosis of cancer patients are known to be closely correlated. HIF-1α, activated by hypoxia conditions or by activation of oncogenes or inactivation of tumor suppressor genes such as pVHL, induces the expression of genes such as hexokinase 2, glucose transporter 1, erythropoietin, IGF-2, endoglin, VEGFA, MMP-2, uPAR, and MDR1, thereby acquiring characteristics such as resistance to apoptosis, increased angiogenesis, increased cell proliferation, and increased cell migration (metastasis) and invasion, which ultimately leads to malignant cancer cells.

In addition, it is known that the active activity of HIF-1 protein promotes tumor growth and interferes with the effectiveness of chemotherapy or radiotherapy, worsening the condition of patients. As such, HIF-1 plays an important role in the growth, proliferation, and malignancy of cancer, especially solid cancer, so research is being actively conducted to develop anticancer drugs targeting HIF-1 (Cancer Res. 62, 4316, 2002; Nat Rev. Drug Discov. 2, 1, 2003; Nat. Rev. Cancer 3, 721-732, 2003).

### [Disclosure]

### [Technical Problem]

Accordingly, the inventors of the present invention researched to develop an anticancer agent targeting HIF-1α, and, as a result, confirmed that steppogenin inhibits the growth and metastasis of cancer cells by suppressing HIF-1α expression, thus completing the present invention.

Therefore, the present invention has been made in view of the above problems, and it is one object of the present invention to provide a pharmaceutical composition for preventing or treating cancer including steppogenin or its pharmaceutically acceptable salt as an active ingredient.

It is another object of the present invention to provide a pharmaceutical composition for inhibiting, preventing or treating cancer metastasis including steppogenin or its pharmaceutically acceptable salt as an active ingredient.

It is still another object of the present invention to provide a food composition for preventing or ameliorating cancer including steppogenin or its nutraceutically acceptable salt as an active ingredient.

However, the technical problems to be achieved by the present invention are not limited to the problems mentioned above, and other problems not mentioned can be clearly understood by a person having ordinary skill in the technical field to which the present invention belongs from the description below.

### [Technical Solution]

In accordance with an aspect of the present invention, the above and other objects can be accomplished by the provision of a pharmaceutical composition for preventing or treating cancer, including steppogenin or its pharmaceutically acceptable salt as an active ingredient.

In accordance with another aspect of the present invention, there is provided a pharmaceutical composition for inhibiting, preventing or treating cancer metastasis, including steppogenin or its pharmaceutically acceptable salt as an active ingredient.

In accordance with still another aspect of the present invention, there is provided a food composition for preventing or ameliorating cancer, including steppogenin or its nutraceutically acceptable salt as an active ingredient.

In an embodiment of the present invention, the cancer is a solid cancer, but is not limited thereto.

In another embodiment of the present invention, the cancer may be selected from the group consisting of colorectal cancer, liver cancer, stomach cancer, breast cancer, colon cancer, bone cancer, pancreatic cancer, lung cancer, glioma, head and neck cancer, metastatic cancer, melanoma, uterine cancer, ovarian cancer, rectal cancer, esophageal cancer, small intestinal cancer, anal cancer, colon cancer, fallopian tube carcinoma, endometrial carcinoma, cervical carcinoma, vaginal carcinoma, vulvar carcinoma, Hodgkin's disease, prostate cancer, bladder cancer, kidney cancer, ureteral cancer, renal cell carcinoma, renal pelvic carcinoma and central nervous system tumor, but is not limited thereto.

In still another embodiment of the present invention, the steppogenin may inhibit the expression of the hypoxia-inducible factor-1 alpha (HIF-1α) gene or reduce the level of protein expressed by the HIF-1α gene, without being limited thereto.

In still another embodiment of the present invention, the composition may inhibit growth of cancer cells, without being limited thereto.

In still another embodiment of the present invention, the composition may further include a chemotherapeutic agent or an immunotherapeutic agent, without being limited thereto.

In accordance with still another aspect of the present invention, there is provided a method of preventing or treating cancer, the method comprising: administering a composition comprising steppogenin or its pharmaceutically acceptable salt to a subject in need thereof.

In accordance with still another aspect of the present invention, there is provided use of a composition including steppogenin or its pharmaceutically acceptable salt for prevention or treatment of cancer.

In accordance with still another aspect of the present invention, there is provided use of steppogenin or its pharmaceutically acceptable salt for the preparation of a medicament for cancer treatment.

In accordance with still another aspect of the present invention, there is provided a method of inhibiting, preventing or treating cancer metastasis, including: administering a composition comprising steppogenin or its pharmaceutically acceptable salt to a subject in need thereof.

In accordance with still another aspect of the present invention, there is provided use of a composition including steppogenin or its pharmaceutically acceptable salt for the inhibition, prevention or treatment of cancer metastasis.

In accordance with yet another aspect of the present invention, there is provided use of steppogenin or its pharmaceutically acceptable salt for the preparation of a medicament for inhibiting cancer metastasis.

### [Advantageous effects]

The steppogenin of the present invention can inhibit the growth of cancer cells by suppressing the expression of HIF-1α that is overexpressed in cancer cells. Therefore, the steppogenin is expected to be used for the prevention, amelioration, or treatment of various solid cancer diseases such as lung cancer, liver cancer, and stomach cancer, and also to be usefully utilized for suppressing the malignancy, recurrence, and metastasis of cancer.

### [Description of Drawings]

FIG. 1 illustrates the results of confirming the cytotoxicity of 70 candidate compounds.
FIG. 2a illustrates the results of confirming the HIF-1α promoter reporter activity of 70 candidate compounds (*p < 0.05, **p < 0.005, and ***p< 0.001 vs. hypoxic control group).
FIG. 2b illustrates the results of confirming whether compounds selected as potential HIF-1α inhibitors among 70 candidate compounds inhibit HIF-1α protein expression (H: hypoxia; *p < 0.05, **p < 0.005, and ***p< 0.001 vs. hypoxic control group; $$p < 0.01 vs. normoxic control group).
FIG. 2c illustrates the results of confirming whether compounds, which inhibit HIF-1α protein expression, among compounds selected as potential HIF-1α inhibitors inhibit DLL4 protein expression (*p < 0.05, **p < 0.005, and ***p< 0.001 vs. VEGF-treated control group; $$p < 0.01 vs. untreated control group).
FIG. 3a illustrates the results of confirming cell viability for steppogenin (H: hypoxia; *p < 0.05, **p < 0.005, and ***p< 0.001 vs. hypoxic control group).
FIG. 3b illustrates the results of confirming the cytotoxicity of steppogenin (H: hypoxia).
FIG. 3c illustrates the results of confirming the HRE-luciferase reporter activity of steppogenin (H: hypoxia; *p < 0.05, **p < 0.005, and ***p< 0.001 vs. hypoxic control group).
FIG. 3d illustrates the results of confirming the IC50 of steppogenin for HIF-1α activity (H: hypoxia).
FIG. 3e illustrates the results of confirming the DLL4-luciferase reporter activity of steppogenin (*p < 0.05, **p < 0.005, and ***p< 0.001 vs. VEGF-treated control group).
FIG. 3f illustrates the results of confirming the IC50 of steppogenin for DLL4 activity.
FIG. 4a illustrates the results of confirming whether steppogenin inhibits HIF-1α protein expression in human embryonic kidney epithelial cells HEK293T, human lung cancer cells A549, and human retinal pigment epithelial cells ARPE19 under hypoxia conditions (H: hypoxia; *p < 0.05, **p < 0.005, and ***p< 0.001 vs. hypoxic control group; $p < 0.05, $$p < 0.01, and $$$p < 0.001 vs. normoxic control group).
FIG. 4b illustrates the results of immunofluorescence analysis to confirm the effect of steppogenin on the intranuclear expression of HIF-1α under hypoxia conditions (NOR: normoxia, HYPO or H: hypoxia).
FIG. 4c illustrates the results of confirming the effect of steppogenin on the mRNA expression of HIF-1α target genes VEGF, GLUT1, CXCR4, and CA9 under hypoxia conditions (H: hypoxia; *p < 0.05, **p < 0.005, and ***p< 0.001 vs. hypoxic control group; $p < 0.05, $$p < 0.01, and $$$p < 0.001 vs. normoxic control group).
FIG. 4d illustrates the results of confirming the effect of steppogenin on the protein levels of HIF-1α target genes VEGF, GLUT1, CXCR4, and CA9 under hypoxia conditions (H: hypoxia; *p < 0.05, **p < 0.005, and ***p< 0.001 vs. hypoxic control group; $p < 0.05, $$p < 0.01, and $$$p < 0.001 vs. normoxic control group).
FIG. 5a illustrates the results of confirming the effect of steppogenin on DLL4, Notch1, and NICD protein levels under VEGF treatment (*p < 0.05, **p < 0.005, and ***p< 0.001 vs. VEGF-treated control group; $p < 0.05, $$p < 0.01, and $$$p < 0.001 vs. untreated control group).
FIG. 5b illustrates the results of immunofluorescence analysis to confirm the effect of steppogenin on DLL4 expression under hypoxia conditions (NOR: normoxia, HYPO or H: hypoxia; *p < 0.05, **p < 0.005, and ***p< 0.001 vs. hypoxic control group; $p < 0.05, $$p < 0.01, and $$$p < 0.001 vs. normoxic control group).
FIG. 5c illustrates the results of confirming the effect of steppogenin on cell migration under hypoxia conditions (NOR: normoxia, HYPO or H: hypoxia; *p < 0.05, **p < 0.005, and
***p< 0.001 vs. hypoxic control group; $p < 0.05, $$p < 0.01, and $$$p < 0.001 vs. normoxic control group).
FIG. 5d illustrates the results of confirming the effect of steppogenin on EA.hy926 (HUVEC) spheroid germination under VEGF treatment (*p < 0.05, **p < 0.005, and ***p< 0.001 vs. VEGF-treated control group; $p < 0.05, $$p < 0.01, and $$$p < 0.001 vs. untreated control group).
FIG. 6a illustrates the results of confirming the effect of steppogenin on tumor growth in tumor animal models (*p < 0.05, **p < 0.005, ***p< 0.001, and ****p < 0.0001 vs. control, the rest are the same).
FIG. 6b illustrates the results of confirming the changes in the body weights of tumor animal models after steppogenin administration.
FIG. 6c illustrates the results of confirming the effect of steppogenin on hypoxic region and HIF-1α expression in tumors of tumor animal models (hypoxic region: green, HIF-1α: red). Scale bar = 100 µm.
FIG. 6d illustrates the results of confirming the effect of steppogenin on angiogenesis through microvessel density analysis using CD34 antibody in tumor animal models (CD34: red).
FIG. 6e illustrates the results of immunofluorescence analysis to confirm the effect of steppogenin on CD3+, CD4+, and CD8+ T cells in tumor animal models.

### [Best Mode]

The present inventors researched to develop an anticancer agent targeting HIF-1α and DLL4 and, as a result, confirmed that steppogenin inhibited the growth and metastasis of cancer cells and angiogenesis by suppressing the expression of HIF-1α and DLL4, thus completing the present invention.

Hereinafter, the present invention is described in detail.

The present invention provides a pharmaceutical composition for preventing or treating cancer including steppogenin or its pharmaceutically acceptable salt as an active ingredient.

As another aspect, the present invention also provides a pharmaceutical composition for inhibiting cancer metastasis including steppogenin or its pharmaceutically acceptable salt as an active ingredient.

In this specification, "steppogenin" may be represented by Formula 1 below, and may have the IUPAC name of (2S)-2-(2,4-dihydroxyphenyl)-5,7-dihydroxy-2,3-dihydrochromen-4-one. In addition, its molecular weight may be 288.25, and its chemical formula may be C₁₅H₁₂O₆. In addition, the steppogenin may be chemically synthesized by a method known in the field to which the present invention belongs, or may be a commercially available material, and may be obtained by extraction or separation, without being limited thereto.

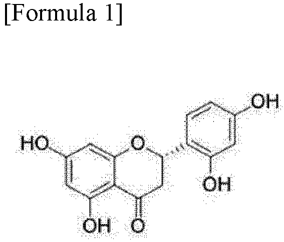

The present invention may also include a pharmaceutically acceptable salt of steppogenin as an active ingredient. In the present invention, the term "pharmaceutically acceptable salt" includes salts derived from pharmaceutically acceptable inorganic acids, organic acids, or bases.

Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. An acid addition salt may be prepared by conventional methods, for example, by dissolving a compound in an excess of aqueous acid solution and precipitating the salt using a water-miscible organic solvent such as methanol, ethanol, acetone or acetonitrile. In addition, the acid addition salt may be prepared by heating an acid or alcohol in an equal molar amount of a compound and water, and then evaporating the mixture to be dried, or by suction-filtering a precipitated salt.

Salts derived from suitable bases may include, but are not limited to, alkali metals such as sodium, potassium, alkaline earth metals such as magnesium, ammonium, and the like. The alkali metals or alkaline earth metal salts may be obtained, for example, by dissolving a compound in an excess amount of an alkali metal hydroxide or alkaline earth metal hydroxide solution, filtering the undissolved compound salt, and then evaporating and drying the filtrate. Here, it is pharmaceutically suitable to prepare a sodium, potassium, or calcium salt as the metal salt, and the corresponding silver salt may be obtained by reacting an alkali metal or alkaline earth metal salt with a suitable silver salt (e.g., silver nitrate).

The content of the steppogenin or its pharmaceutically acceptable salt in the composition of the present disclosure may be appropriately adjusted depending on the symptoms of diseases, the degree of progression of the symptoms, the conditions of patients, etc., for example, 0.0001 to 99.9% by weight or 0.001 to 50% by weight based on the total weight of the composition, but is not limited thereto. The content ratio is a value based on the dry amount from which the solvent is removed.

According to an embodiment of the present invention, the steppogenin can not only inhibit the expression of the hypoxia-inducible factor-1 alpha (HIF-1α) gene or reduce the level of the protein expressed by the HIF-1α gene, but also inhibit the expression of the VEGF, GLUT1, CXCR4, and CA9 genes, which are target genes of HIF-1α, or reduce the level of the protein expressed by the VEGF, GLUT1, CXCR4, and CA9 genes. In addition, steppogenin can inhibit the growth and metastasis of cancer cells by inhibiting angiogenesis and cell migration.

In the present invention, "expression" includes all steps in the process of making a gene into a protein in vitro or within cells, for example, transcription of a gene into mRNA and translation of mRNA into a protein.

In the present invention, "cancer" refers to or denotes a physiological condition typically characterized by uncontrolled cell growth in mammals. In the present invention, cancer to be prevented, ameliorated or treated may be a solid tumor formed by a lump of abnormally grown cells in a solid organ, and depending on the location of the solid organ, may be colon cancer, liver cancer, stomach cancer, breast cancer, colon cancer, bone cancer, pancreatic cancer, lung cancer, glioma, head and neck cancer, metastatic cancer, melanoma, uterine cancer, ovarian cancer, rectal cancer, esophageal cancer, small intestine cancer, anal cancer, colon cancer, fallopian tube carcinoma, endometrial carcinoma, cervical carcinoma, vaginal carcinoma, vulvar carcinoma, Hodgkin's disease, prostate cancer, bladder cancer, kidney cancer, ureteral cancer, renal cell carcinoma, renal pelvic carcinoma, central nervous system tumor, etc. and preferably, may be lung cancer, but is not limited thereto.

In the present invention, the term "metastasis" refers to a state in which a malignant tumor has spread to other tissues away from the organ where it developed. As a malignant tumor that started in one organ progresses, it spreads from the original site of the organ to other tissues. This spreading from the original site to other tissues can be called metastasis. Metastasis can be said to be a phenomenon accompanying the progression of malignant tumors. Metastasis can occur as malignant tumor cells proliferate and acquire new genetic characteristics as the cancer progresses. When tumor cells that have acquired new genetic traits invade blood vessels and lymph nodes, circulate through the blood and lymph, and settle and proliferate in other tissues, metastasis can occur.

In the present invention, the composition may additionally include, but is not limited to, a chemotherapeutic agent or an immunotherapeutic agent.

In this specification, "active ingredient" means an ingredient that can exhibit the intended activity together with a carrier or the like that exhibits the intended activity alone or is inactive in itself.

The pharmaceutical composition according to the present invention may further include a suitable carrier, excipient and diluent commonly used in the preparation of a pharmaceutical composition. The excipient may be, for example, one or more selected from the group consisting of diluents, binders, disintegrants, glidants, adsorbents, moisturizers, film-coating materials, and controlled-release additives.

The pharmaceutical composition according to the present invention may be formulated and used in the form of external preparations such as powders, granules, sustained-release granules, enteric-coated granules, liquids, eye drops, ellipses, emulsions, suspensions, alcohols, troches, aromatic waters, limonades, tablets, sustained-release tablets, enteric-coated tablets, sublingual tablets, hard capsules, soft capsules, sustained-release capsules, enteric capsules, pills, tinctures, soft extracts, dry extracts, fluid extracts, injections, capsules, irrigation solutions, warnings, lotions, pastes, sprays, inhalants, patches, sterile injection solutions, or aerosols, according to conventional methods, and the external preparations may be formulated and used in the form of external preparations such as creams, gels, patches, sprays, ointments, warnings, lotions, liniments, pastes, or cataplasmas.

Examples of a carrier, an excipient and a diluent which may be included in the pharmaceutical composition according to the present invention may include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like.

When formulating, a commonly used diluent or excipient such as a filler, a bulking agent, a binder, a wetting agent, a disintegrant, or a surfactant is used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate, and Primojel; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ionexchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, sodium carboxymethylcellulose, and the like may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote (SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs), and tegester triglyceride matter (TG-95, MA, 57) may be used.

Solid preparations for oral administration include tablets, pills, powders, granules, capsules, and the like, and such solid preparations are formulated by mixing the extract with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like.

Preparations for parenteral administration include an aqueous sterile solution, a non-aqueous solvent, a suspension, an emulsion, a freeze-dried preparation, and a suppository. As the non-aqueous solvent and the suspension, propylene glycol, polyethylene glycol, vegetable oil such as olive oil, and an injectable ester such as ethyl oleate, and the like may be used.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including the types of diseases of patients, the severity of the disease, the activity of drugs, sensitivity to drugs, administration time, administration route, excretion rate, treatment period, and simultaneously used drugs, and factors well known in other medical fields.

The pharmaceutical composition according to the present disclosure may be administered as an individual therapeutic agent, may be administered in combination with other therapeutic agents, may be administered sequentially or simultaneously with conventional therapeutic agents, and may be administered once or multiple times. In consideration of all of the above factors, it is important to administer an amount capable of obtaining the maximum effect with a minimum amount without side effects, which can easily be determined by those skilled in the art to which the present disclosure pertains.

The pharmaceutical composition of the present disclosure may be administered to a subject by various routes. All modes of administration may be considered, for example, oral administration, subcutaneous injection, intraperitoneal administration, intravenous injection, intramuscular injection, paraspinal space (intrathecal) injection, sublingual administration, buccal administration, rectal insertion, vaginal insertion, ocular administration, otic administration, nasal administration, inhalation, spray through the mouth or nose, dermal administration, transdermal administration, etc.

The pharmaceutical composition of the present disclosure is determined according to the type of drug as an active ingredient along with several related factors such as the disease to be treated, route of administration, patient's age, sex, weight, and severity of the disease.

In addition, the present invention provides a method of preventing or treating cancer including a step of administering a composition including steppogenin or its pharmaceutically acceptable salt to a subject in need thereof.

In addition, the present invention provides use of a composition including steppogenin or its pharmaceutically acceptable salt for the prevention or treatment of cancer.

In addition, the present invention provides use of steppogenin or its pharmaceutically acceptable salt for the preparation of a medicament for the treatment of cancer.

In addition, the present invention provides a method of inhibiting, preventing or treating cancer metastasis including a step of administering a composition including steppogenin or its pharmaceutically acceptable salt to a subject in need thereof.

In addition, the present invention provides use of a composition including steppogenin or its pharmaceutically acceptable salt for the inhibition, prevention or treatment of cancer metastasis.

In addition, the present invention provides use of steppogenin or its pharmaceutically acceptable salt for the preparation of a drug for inhibiting cancer metastasis.

In the present invention, the term "subject" refers to a subject in need of treatment for a disease, and more specifically, may be a mammal of humans or non-human primates, mice, rats, dogs, cats, horses, cattle, etc., but is not limited thereto.

In the present invention, the term "administration" refers to provision of a predetermined composition of the present invention to a subject by any suitable method.

In the present invention, the term "prevention" refers to all actions that inhibit or delay the onset of a target disease; and the term "treatment" refers to all actions that improve or beneficially change a target disease and metabolic abnormalities thereof by the administration of the pharmaceutical composition according to the present invention.

As another aspect, the present invention provides a food composition for preventing or ameliorating cancer, including steppogenin or its nutraceutically acceptable salt as an active ingredient.

In the present invention, the term "nutraceutically acceptable salt" includes salts derived from nutraceutically acceptable organic acids, inorganic acids, or bases.

In the present invention, "amelioration" means any action that reduces a parameter related to a target disease, such as the degree of symptoms, by administering a composition according to the present invention. Here, the food composition may be used simultaneously or separately with drugs for the treatment of cancer before or after the onset of cancer for the prevention or amelioration of cancer.

When the steppogenin or its nutraceutically acceptable salt of the present invention is used as a food additive, the steppogenin or its nutraceutically acceptable salt may be added as is or used together with other foods or food ingredients, and may be used appropriately according to existing methods. The mixing amount of an active ingredient may be appropriately determined according to the purpose of use (prevention, health maintenance or therapeutic treatment). In general, when manufacturing food or beverage, the steppogenin or its nutraceutically acceptable salt of the present invention may be added in an amount of 15% by weight or less, or 10% by weight or less, to the raw material. However, for long-term consumption for health and hygiene purposes or health control purposes, the amount may be below the above range, and an active ingredient may be used in amounts above the above range as there is no problem in terms of safety.

There is no special limitation on the types of the above foods. Examples of foods to which the substance can be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and include all healthy functional foods in the conventional sense.

A healthy drink composition according to the present invention may contain various flavoring agents or natural carbohydrates as additional ingredients, like conventional drinks. The natural carbohydrates are monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extracts, or synthetic sweeteners such as saccharin and aspartame may be used. The proportion of the natural carbohydrates is generally about 0.01 to 0.20 g, or about 0.04 to 0.10 g per 100 mL of the composition of the present invention.

In addition to the above, the composition of the present invention may contain various nutrients, vitamins, electrolytes, flavoring agents, coloring agents, pectic acid and its salts, alginic acid and its salts, organic acids, protective colloid thickeners, pH regulators, stabilizers, preservatives, glycerin, alcohol, carbonating agents used in carbonated beverages, etc. In addition, the composition of the present invention may contain fruit pulp for the production of natural fruit juice, fruit juice drinks and vegetable drinks. These components may be used independently or in combination. The proportion of these additives is not very important, but is usually selected in the range of 0.01 to 0.20 parts by weight per 100 parts by weight of the composition of the present invention.

In the present invention, the food composition may be a healthy functional food composition, but is not limited thereto.

In this specification, "healthy functional food" is the same term as food for special health use (FoSHU), and means food with high medical and healthcare effects which is processed to efficiently exhibit bioregulatory functions in addition to nutritional supply. The food may be manufactured in various forms such as tablets, capsules, powders, granules, liquids, and pills to obtain useful effects in preventing or ameliorating cancer or cancer metastasis.

The healthy functional food of the present invention may be prepared by a method commonly used in the art, and during the preparation process, raw materials and ingredients commonly added in the art may be added to the food. In addition, unlike general drugs, it has the advantage of not having side effects that may occur when taking drugs for a long time because it uses food as a raw material, and it can be highly portable.

### [Mode for Carrying Out the Invention]

Hereinafter, preferred examples are presented to help understand the present invention. However, the following examples are provided only to help understand the present invention more easily, and the contents of the present invention are not limited by the following examples.

### [Experimental examples]

### Experimental Example 1. Materials

The 70 compounds used (isolated from various natural herbs) were provided by Professor Baek Nam-in of Kyung Hee University's nature compound library (College of Life Science, Kyung Hee University, Korea). Before the experiment, the 70 compounds were randomly numbered, and each compound was dissolved in dimethyl sulfoxide (DMSO) to prepare a 10 mM stock concentration. Information on the 70 compounds used was not disclosed except for steppogenin (compound #57), and tanespimycin (17-AAG) was purchased from Selleckchem (Pittsburgh, PA, US).

### Experimental Example 2. Cell culture and hypoxic conditions

Human embryonic kidney epithelial cells (HEK293T; KCLB, Seoul, Korea) and human umbilical vein EC (HUVEC; EA.hy926; ATCC, Manassas, VA, USA) were cultured in Dulbecco's modified Eagle's medium (Hyclon, Logan, UT, USA), human lung cancer cells (A549; ATCC) were cultured in RPMI-1640 (Hyclon), and human retinal pigment epithelial cells (ARPE-19) were cultured in DME/F-12 media (Hyclon) containing 1% antibiotics (100-units/mL penicillin and 100-mg/mL streptomycin; Invitrogen, Carlsbad, CA, USA) and 10% fetal bovine serum (FBS). HUVEC (passaged 2 to 8 times) were grown on gelatin-coated (0.1% gelatin; Sigma-Aldrich) culture dishes containing EBM-2 (Lonza, Basel, Switzerland) supplemented with EGM-2 Kit, 10% FBS, and 1% antibiotics. All cells were cultured under normoxic conditions (5% CO₂ and 95% air) and exposed to hypoxic conditions (5% CO₂, 1% O₂, and 94% N₂) in hypoxia chambers (ASTEC, Fukuoka, Japan).

### Experimental Example 3. Animal model

Six-week-old adult male C57BL/6J mice (SLC, Japan) were used and housed in a specific pathogen-free facility with an individual ventilation system at Kyungpook National University. Animal handling and experimental procedures were performed in accordance with the Animal Care and Use Guidelines (Protocol Numbers: KNU 2014-0189 and KNU 2017-0145) notified by the Animal Hospital Ethics Committee of Kyungpook National University.

### Experimental Example 4. Cell counting kit-8 assay

HEK293T cells were seeded at a density of 5 × 10³ cells/well in 96-well plates and cultured at 37°C for 24 h. After 24 hours, the natural compounds were diluted in the medium according to the concentration and replaced. Cell viability was determined after exposure to hypoxic conditions for 24 h using the Cell Counting Kit-8 (DOJINDO, Rockville, MD, USA) according to the manufacturer's instructions, and measured using a microplate reader (Infinite M200 Pro; TECAN, Mannedorf, Switzerland) at 450 nm.

### Experimental Example 5. Dual-luciferase assay

To perform the HRE-luciferase assay, HEK293T cells were seeded in a 96-well plate at a density of 5×10³ cells/well and cultured at 37°C. Next, cells were co-transfected with pGL3-HRE-luciferase plasmid and pRL-SV40 Renilla plasmid containing 5 copies of HRE of human VEGF gene using a vivamagic transfection reagent (Vivagen, Seongnam, Korea) (Kwon et al., 2015). After 24 h, cells were pretreated with various concentrations of natural compounds for 1 h and then incubated under hypoxia conditions for 24 h. To perform the DLL4-luciferase assay, EA.hy926 cells were seeded in 24-well plates at a density of 4 × 10⁴ cells/well and cultured at 37 °C for 24 h. Cells were co-transfected with pGL3-DLL4 luciferase and pRL-SV40 Renilla plasmids using Lipofectamine 2000 (Invitrogen) and cultured at 37°C for 24 h. Next, the cells were incubated with various concentrations of natural compounds for 1 h and then incubated for 24 h in the presence or absence of VEGF (10 ng/mL). The luminescence of HRE- and DLL4-promoter activities was detected using a microplate reader and the Dual-Luciferase Assay Kit (Promega, Madison, WI, USA) according to the manufacturer's instructions. The luciferase activity of the natural compounds was normalized to Renilla luciferase activity.

### Experimental Example 6. Immunofluorescence staining

Cells were seeded at a density of 2 × 10⁴ - 3 × 10⁴ cells/well on coverslips of 4-well plates and cultured for 24 h. For HIF-1α staining, A549 cells were treated with various compounds and incubated under normoxia or hypoxia conditions for 4 h. For Ki-67 and DLL4 staining, EA.hy926 cells were treated with the same compounds and maintained under VEGF (20 ng/mL) treatment. Next, the cells were fixed with 4% paraformaldehyde and permeabilized with 1% Triton X-100. The fixed cells were blocked with phosphate-buffered saline (PBS) containing 3% bovine serum albumin (BSA) and then incubated with primary antibodies overnight. After incubation, the cells were incubated with secondary antibodies for 1 hour and their nuclei were stained using DAPI. The stained cells were visualized using a confocal microscope (Leica TCS SP5 II Dichroic/CS, Leica, Germany).

### Experimental Example 7. Western blot analysis

Proteins were extracted from cultured cells using RIPA lysis buffer (Thermo Scientific, Waltham, MA, USA). The protein extracts were separated using SDS-PAGE and transferred to nitrocellulose membranes (Whatman, Maidstone, England). The membranes were blocked with 5% skim milk in Tris-buffered saline containing 0.1% Tween-20 for 1 hour at room temperature. After blocking, the membranes were incubated with specific primary antibodies overnight at 4°C, and then incubated with secondary antibodies for 1 hour at room temperature. Proteins were detected using the Enhanced Chemiluminescence Kit (Bio-Rad, Hercules, CA, USA) according to the manufacturer's instructions. Primary antibodies against HIF-1α (BD Biosciences, San Diego, CA, USA), VEGF (Santa Cruz Biotechnology, Santa Cruz, CA, USA), CXCR4 (Abcam, Cambridge, UK), CA9 (Abcam), DLL4 (Santa Cruz Biotechnology), NOTCH1 (Thermo Scientific), and β-actin (Santa Cruz Biotechnology) were used.

### Experimental Example 8. Lactate dehydrogenase cytotoxicity assay

HEK293T cells were seeded at 2 × 10³ cells/well in 96-well plates and cultured at 37 °C for 24 h. Next, the cells were treated with various concentrations of steppogenin for 24 h under hypoxia conditions. Lactate dehydrogenase (LDH) release values were evaluated using the Cytotoxicity LDH Assay Kit-WST (DOJINDO) according to the manufacturer's instructions. Cytotoxicity was measured using a microplate reader (Infinite M200 Pro; TECAN) at 490 nm.

### Experimental Example 9. RNA isolation and quantitative reverse-transcription polymerase chain reaction

Total RNA was isolated using TRIzol Reagent (Invitrogen). The isolated RNA was used to synthesize cDNA using ReverTra Ace qPCR RT Master Mix (TOYOBO, Osaka, Japan) according to the manufacturer's protocol. The synthesized cDNA was mixed with gene-specific primers. Amplification real-time polymerase chain reaction (PCR) was performed using SYBR Green PCR Master Mix (Applied Biosystems, CA, USA). The heat cycle proceeded as follows: 50 °C for 2 min, 95 °C for 10 min, 40 cycles of denaturation at 95 °C for 15 s, and annealing at 60 °C for 1 min. Mean cycle threshold values were used to calculate mRNA expression with normalization to β-actin as an internal control. The sequences of specific primers for real-time PCR are shown in Table 1.

**[Table 1]**

| Gene | | Primer sequences |
|---|---|---|
| VEGF | Forward | 5'-ACCATGAACTTTCTGCTC-3' (SEQ No: 1) |
| | Reverse | 5'-GGACGGCTTGAAGATATA-3' (SEQ No: 2) |
| GLUT1 | Forward | 5'-ATTGGCTCCGGTATCGTCAA-3' (SEQ No: 3) |
| | Reverse | 5'-ATGGCCACGATGCTCAGATA-3' (SEQ No: 4) |
| CXCR4 | Forward | 5'-CTGAGAAGCATGACGGACAA-3' (SEQ No: 5) |
| | Reverse | 5'-CGCCAACATAGACCACCTTT-3' (SEQ No: 6) |
| CA9 | Forward | 5'-GTGTAGTCAGAGACCCCTCA-3' (SEQ No: 7) |
| | Reverse | 5'-GGAAGAAAACAGTGCCTATG-3' (SEQ No: 8) |
| β-actin | Forward | 5'-GACTACCTCATGAAGATC-3' (SEQ No: 9) |
| | Reverse | 5'-GATCCACATCTGCTGGAA-3' (SEQ No: 10) |

### Experimental Example 10. Wound-healing migration assay

EA.hy926 cells were seeded at a density of 4 × 10⁴ cells/well in 12-well plates and incubated for 24 hours. The cells were starved overnight in a serum-free medium and treated with mitomycin C (0.5 µg/mL) for 1 h. When confluent, a wound was made with a pipette tip and the cells were washed with a serum-free medium to remove cell debris. Next, the plates were treated with a conditioned medium collected from HEK293T cell cultures under hypoxia conditions treated with steppogenin. Wound closure was monitored and photographs were taken at 0 h and 8 h. Cell migration was measured by calculating the wound area between 0 h and 8 h.

### Experimental Example 11. Spheroid sprouting assay

EA.hy 926 cell suspension (5×10³ cells) was distributed on the lid of a 100 mm dish using a hanging-drop method and maintained for 24 hours. The next day, spheroids were harvested and mixed with Methocel stock solution containing 20% FBS. Next, spheroids containing Methocel solution were seeded in 24-well plates. After incubation at 37°C for 30 minutes, VEGF or steppogenin was added to the polymerized Methocel solution and incubated for 2 days. Finally, germinated endothelial cells (ECs) were calculated and displayed graphically.

### Experimental Example 12. Development of tumor models and therapeutic regimens

To manufacture Lewis lung carcinoma (LLC)-allografted tumor models, LLC cell suspensions (5 × 10⁵ cells in 200 µL of serum-free culture medium) were subcutaneously transplanted into the dorsal flank area of 6-week-old male C57BL/6J mice. After inoculating the LLC cells, the mice were administered 17-AAG (25 mg/kg) or steppogenin (2 mg/kg) via intraperitoneal injection at various time points. Next, tumor volumes were measured according to the following formula: V = 0.5 × A × B², where A and B represent the longest and shortest dimensions, respectively. Finally, the mice were anesthetized and tissues were collected for further analysis.

### Experimental Example 13. Immunohistochemical and histological analyses

For immunohistochemical analysis, collected tumor or tissue samples were fixed in 4% paraformaldehyde and embedded in tissue-freezing medium (Leica, Wetzlar, Germany). Frozen blocks were cut into 10 µm sample sections, blocked with 3% BSA in PBST (0.3% Triton X-100 in PBS), and incubated overnight at 4 °C with appropriate primary antibodies. Primary antibodies were as follows: anti-CD34 (Boster Bio, CA, USA), anti-DLL4 (R&D Systems, MN, USA), anti-pimonidazole (Hypoxyprobe-1, HPI, MA, USA), anti-CD3 (Abcam), anti-CD4 (Invitrogen), and anti-CD8 (Invitrogen). After several washes, the samples were incubated with recommended fluorophore-tagged secondary antibodies for 1 hour at room temperature. Next, cell nuclei were stained using Antifade Mounting Medium containing DAPI (Vector Laboratories, Burlingame, CA). Immunofluorescence images were acquired using a Zeiss confocal microscope (Carl Zeiss, Germany). Next, pimonidazole hydrochloride (60 mg/kg; Hypoxyprobe-1, HPI) was injected intravenously through the tail vein of mice and anesthetized 90 minutes later. Finally, tumor tissues were collected for further analysis.

### Experimental Example 14. Statistical analysis

All data are expressed as the mean ± standard deviation (SD) of at least three samples, and a P value <0.05 was considered statistically significant. Data were analyzed using Student's t test and ANOVA.

### [Examples]

### Example 1. Screening of potential HIF-1α and DLL4 inhibitors in 70 natural compounds

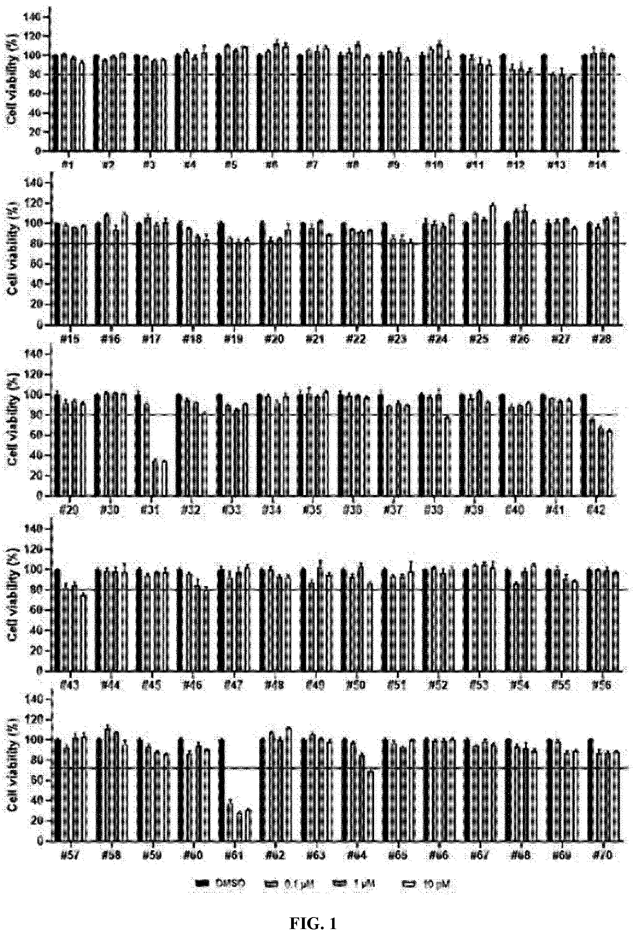

To test the inhibitory activity of 70 natural compounds on HIF-1 and DLL4 activity, their cytotoxic effects on immortalized normal cells were first tested under hypoxia conditions. In particular, under these conditions, HEK293T cells were treated with compounds at various concentrations (0.1 to 10 µM). As a result, two natural compounds (#31 and #61) with low cell viability in hypoxia, as shown in FIG. 1, were excluded from further analysis.

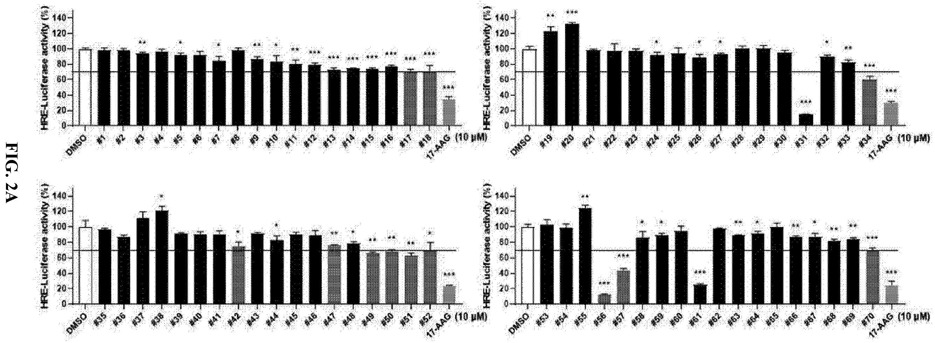

To select potential HIF-1α inhibitors, the effects of 70 natural compounds were tested on HIF-1α promoter reporter activity using a dual-luciferase assay. As a result, as shown in FIG. 2a, 12 natural compounds (#17, #18, #34, #42, #47, #49, #50, #51, #52, #56, #57, and #70) showed <70% HRE-luciferase activity compared to the hypoxic vehicle (DMSO) control group, and were selected as potential HIF-1α inhibitors.

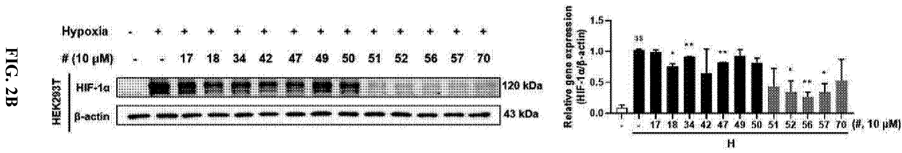

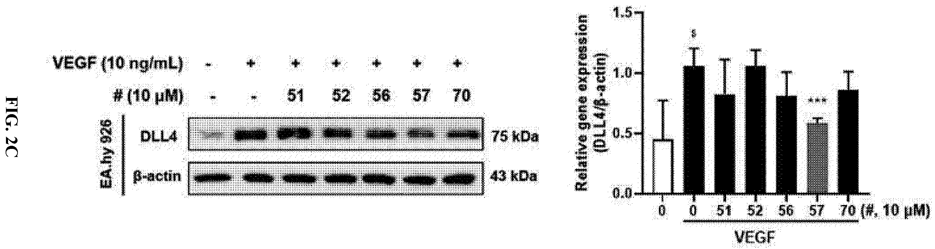

To determine the effect of the selected natural compounds on HIF-1α protein expression, Western blotting was performed on HEK293T cells. As a result, as shown in FIG. 2b, five natural compounds (#51, #52, #56, #57, and #70) inhibited HIF-1α protein expression. Since DLL4 expression in tip ECs (endothelial cells) is induced by VEGF (Blanco and Gerhardt, 2013), simultaneous targeting of HIF-1α in hypoxic tumor cells and DLL4 in vascular ECs effectively inhibited tumor growth and angiogenesis. Accordingly, the effect of five compounds with HIF-1α inhibitory activity on DLL4 reporter activity in ECs was also investigated. As a result, as shown in FIG. 2c, the DLL4 protein level and DLL4 luciferase activity induced by VEGF were significantly suppressed by treatment with Compound #57. Therefore, Compound #57 was selected as a potential inhibitor of HIF-1α and DLL4.

### Example 2. HIF-1α and DLL4 inhibitory activity of steppogenin

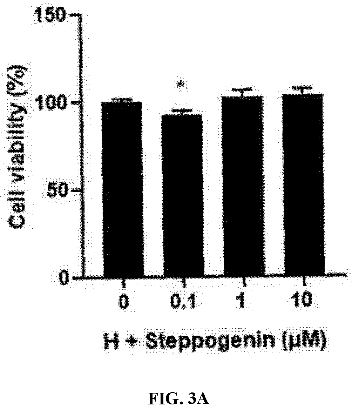

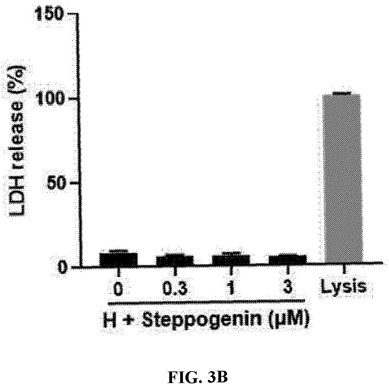

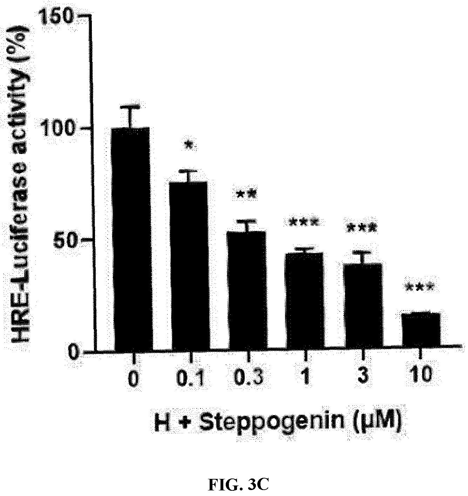

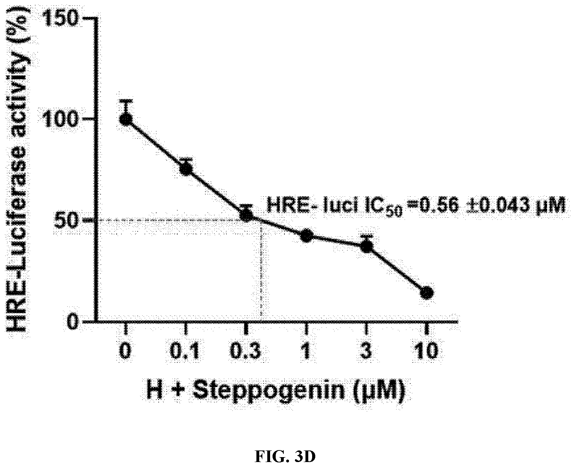

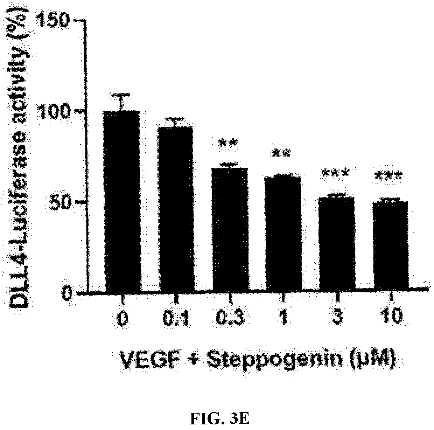

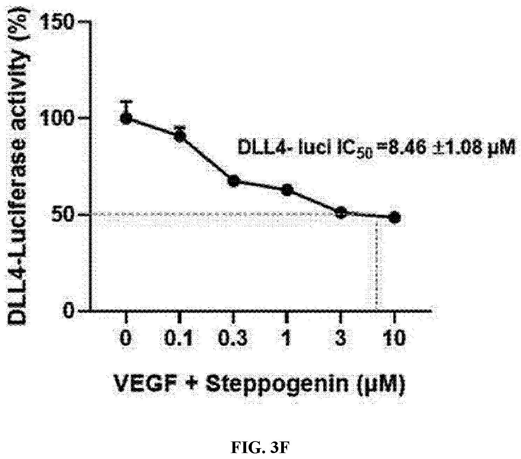

Natural Compound #57 was identified as steppogenin extracted from the root bark of Morus alba L. Additional tests showed that dose-dependent cell viability and cytotoxicity under hypoxia conditions were not altered by steppogenin treatment compared to vehicle treatment, as shown in FIGS. 3a and 3b. However, as shown in FIG. 3c, HRE-luciferase reporter activity was significantly inhibited by 0.1 to 10 µM steppogenin in a dose-dependent manner. In addition, as shown in FIG. 3d, the half-maximal inhibitory concentration (IC50) for HIF-1α activity was 0.56 ± 0.043 µM. DLL4-luciferase reporter activity was also significantly reduced by 0.3 to 10.0 µM steppogenin, as shown in FIG. 3e. As shown in FIG. 3f, the IC50 for DLL4 activity was 8.46 ± 1.08 µM in EA.hy926 cells.

### Example 3. Steppogenin inhibits HIF-1α expression in cancer cells under hypoxic conditions

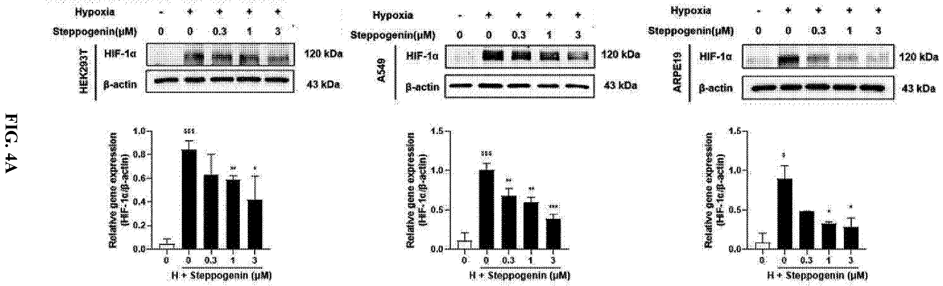

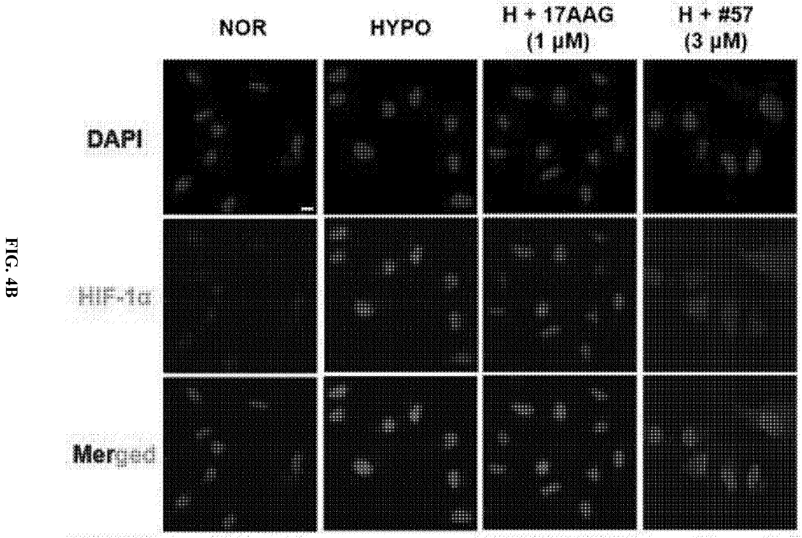

As shown in FIG. 4a, steppogenin significantly inhibited HIF-1α protein levels in a dose-dependent manner in human embryonic kidney epithelial cells HEK293T, human lung cancer cells A549, and human retinal pigment epithelial cells ARPE19, under hypoxia conditions (maximum dose: 3 µM). As shown in FIG. 4b, immunofluorescence analysis results showed that intranuclear expression of HIF-1α increased, compared to a normoxia control, under hypoxia conditions. However, similar to 17-AAG treatment, steppogenin (#57) treatment decreased the intranuclear expression of HIF-1α under hypoxia conditions.

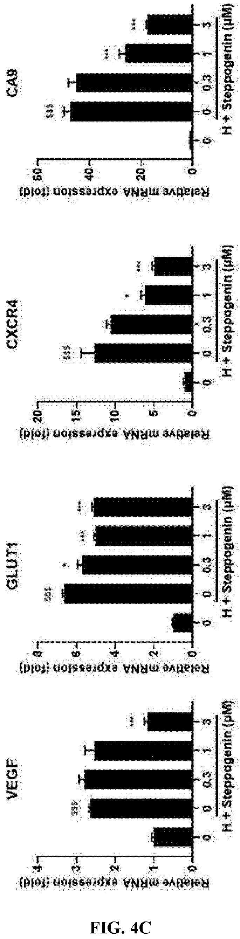

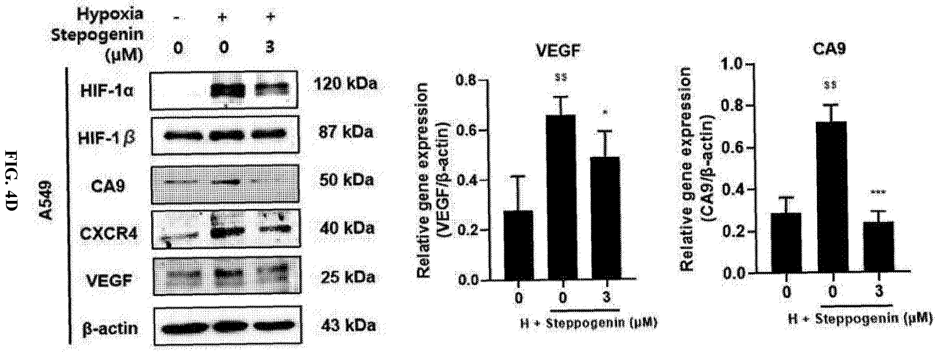

The transcription factor HIF-1α can overcome low oxygen levels or regulate the expression of various target genes involved in angiogenesis, cell survival, metastasis, and glucose metabolism (Semenza, 2007). To confirm the inhibitory effect of steppogenin on HIF-1α activity, the mRNA levels of HIF-1α target genes were examined using real-time qRT-PCR. As a result, steppogenin inhibited the mRNA expression of VEGF, GLUT1, CXCR4, and CA9 under hypoxia conditions, as shown in FIG. 4c. Similarly, as shown in FIG. 4d, steppogenin suppressed the protein levels of VEGF, CXCR4, and CA9, compared to the levels detected in the vehicle control group, under hypoxia conditions. Therefore, it was confirmed that steppogenin inhibited HIF-1α activity and the expression of its target genes.

### Example 4. Stepogenin inhibits DLL4 activation and sprouting angiogenesis in ECs

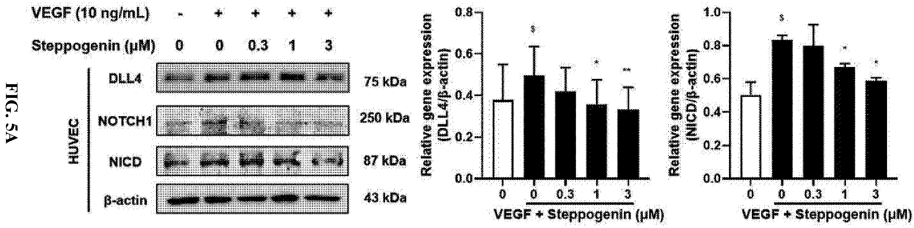

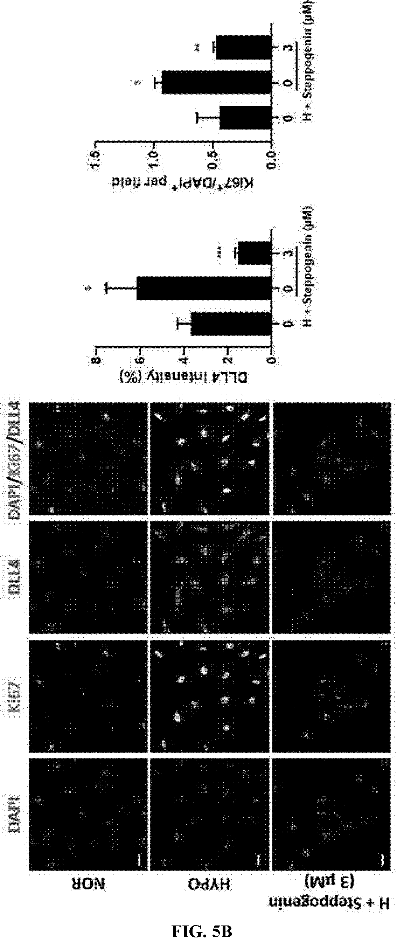

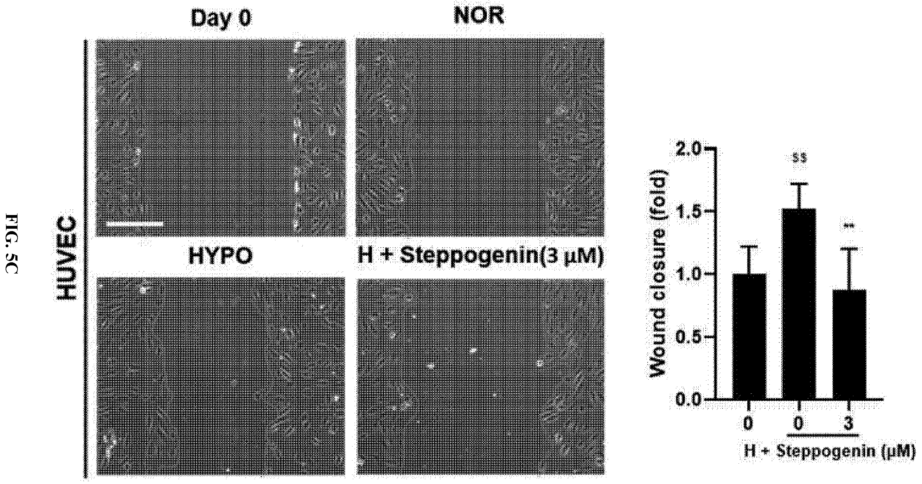

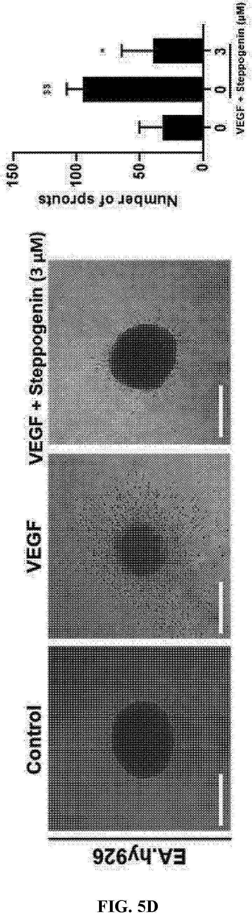

Steppogenin dose-dependently reduced VEGF-induced DLL4, Notch1, and NICD protein levels in HUVECs, as shown in FIG. 5a. Because the DLL4-NOTCH signaling pathway in ECs is important for tip and stalk cell fate during sprouting angiogenesis, the angiogenic efficacy of steppogenin in vitro was evaluated. Interestingly, immunofluorescence analysis showed that DLL4 expression in ECs was significantly increased under hypoxia conditions, as shown in FIG. 5b. However, steppogenin treatment dramatically suppressed DLL4 expression under these conditions. The level of Ki-67, a cell proliferation marker, increased under hypoxia conditions but decreased by steppogenin treatment. Further analysis of HUVECs revealed that the number of migrating cells and wound closure were significantly reduced in the presence of steppogenin under hypoxia conditions, as shown in FIG. 5c. In addition, the germination ability (determined by counting the number of germinations) in 3D EC spheroid cultures was increased by VEGF, but significantly inhibited by steppogenin treatment, as shown in FIG. 5d. Therefore, steppogenin was found to act as an inhibitor of HIF-1α and DLL4 and inhibit angiogenesis in vitro.

### Example 5. Steppogenin inhibits tumor growth and angiogenesis

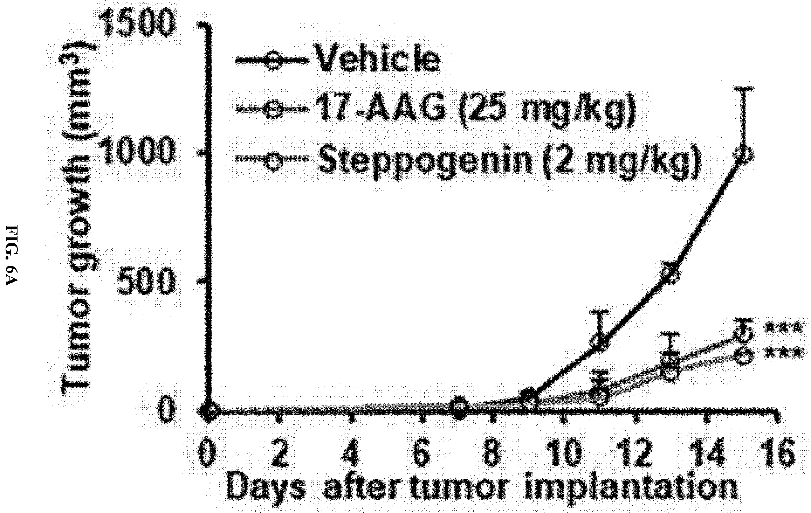

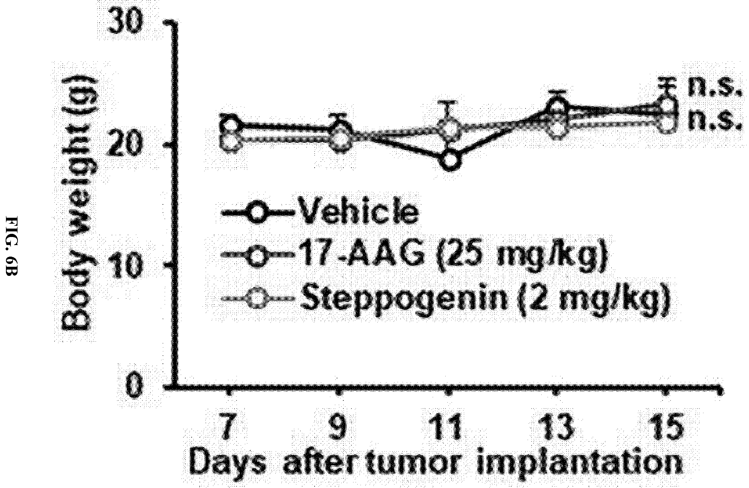

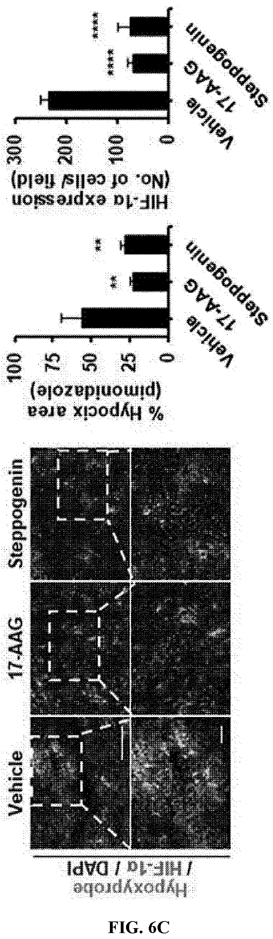

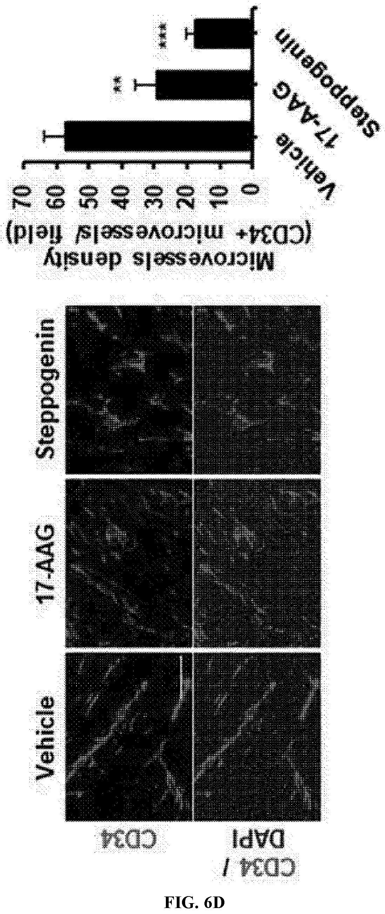

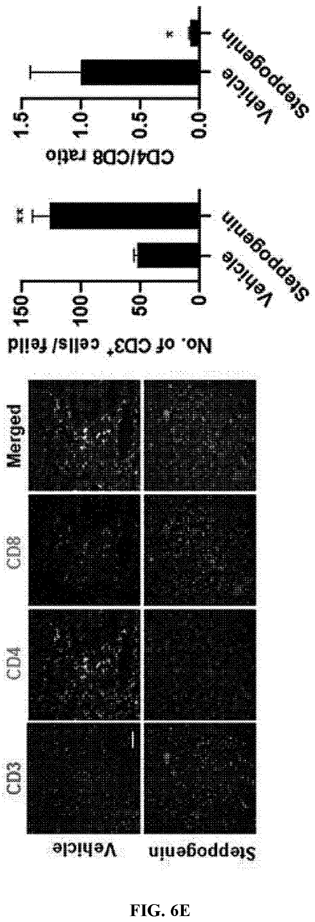

To test the in-vivo effects of steppogenin, LLC allograft tumor models were treated with 17-AAG (25 mg/kg), steppogenin (2 mg/kg), or vehicle (control) on days 7, 9, 11, 13, and 15 after inoculation with the LLC cell suspension of Experimental Example 12. As a result, both 17-AAG and steppogenin significantly inhibited tumor growth, as shown in FIG. 6a. However, there was no significant difference in the average body weights between the groups during the administration period, as shown in FIG. 6b. As shown in FIG. 6c, immunohistochemical staining results showed that the hypoxic region and HIF-1α expression level in tumors were significantly reduced by steppogenin or 17-AAG treatment, compared to the effect in the control group. In addition, microvessel density analyzed using CD34 antibody (a tip-cell marker of tumor tissue) was significantly reduced by steppogenin, compared to that observed in control tissue, as shown in FIG. 6d. Abnormal vascular structures create a hypoxic microenvironment that polarizes inflammatory cells toward immunosuppression (Palazon et al., 2012) (Palazon et al., 2012). To determine whether steppogenin induces vascular normalization, T-cell infiltration in tumor tissues was examined. As a result, steppogenin significantly increased the infiltration of CD3+ T cells and decreased the ratio of cytotoxic (CD8+) to regulatory T (CD4+) cells, as shown in FIG. 6e. These results imply that steppogenin inhibits tumor growth by normalizing tumor blood vessels through the suppression of HIF-1α and EC sprouting activity in tumor tissues.

The aforementioned description of the present invention is provided by way of example and those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention.

### [Industrial Applicability]

The steppogenin of the present invention can inhibit the growth of cancer cells by suppressing the expression of HIF-1α, which is overexpressed in cancer cells, and therefore can be used for the prevention, amelioration, or treatment of various solid cancer diseases such as lung cancer, liver cancer, and stomach cancer. In addition, it can be usefully utilized to suppress the malignancy, recurrence, and metastasis of cancer, thus having industrial applicability.

## Claims

1. A pharmaceutical composition for preventing or treating cancer, comprising steppogenin or its pharmaceutically acceptable salt as an active ingredient.

2. The pharmaceutical composition according to claim 1, wherein the cancer is a solid cancer.

3. The pharmaceutical composition according to claim 1, wherein the cancer is selected from the group consisting of colorectal cancer, liver cancer, stomach cancer, breast cancer, colon cancer, bone cancer, pancreatic cancer, lung cancer, glioma, head and neck cancer, metastatic cancer, melanoma, uterine cancer, ovarian cancer, rectal cancer, esophageal cancer, small intestinal cancer, anal cancer, colon cancer, fallopian tube carcinoma, endometrial carcinoma, cervical carcinoma, vaginal carcinoma, vulvar carcinoma, Hodgkin's disease, prostate cancer, bladder cancer, kidney cancer, ureteral cancer, renal cell carcinoma, renal pelvic carcinoma and central nervous system tumor.

4. The pharmaceutical composition according to claim 1, wherein the steppogenin inhibits expression of the hypoxia-inducible factor-1 alpha (HIF-1α) gene or reduces a level of protein expressed by the HIF-1α gene.

5. The pharmaceutical composition according to claim 1, wherein the composition inhibits growth of cancer cells.

6. The pharmaceutical composition according to claim 1, wherein the composition further comprises a chemotherapeutic agent or an immunotherapeutic agent.

7. A pharmaceutical composition for inhibiting cancer metastasis, comprising steppogenin or its pharmaceutically acceptable salt as an active ingredient.

8. A food composition for preventing or ameliorating cancer, comprising steppogenin or its nutraceutically acceptable salt as an active ingredient.

9. A method of preventing or treating cancer, the method comprising: administering a composition comprising steppogenin or its pharmaceutically acceptable salt to a subject in need thereof.

10. Use of a composition including steppogenin or its pharmaceutically acceptable salt for prevention or treatment of cancer.

11. Use of steppogenin or its pharmaceutically acceptable salt for the preparation of a medicament for cancer treatment.

12. A method of inhibiting cancer metastasis, the method comprising: administering a composition comprising steppogenin or its pharmaceutically acceptable salt to a subject in need thereof.

13. Use of a composition comprising steppogenin or its pharmaceutically acceptable salt for inhibiting cancer metastasis.

14. Use of steppogenin or its pharmaceutically acceptable salt for preparation of a medicament for inhibiting cancer metastasis.
